# EUROPEAN PATENT APPLICATION

(11) **EP 3 584 608 A1**
(43) Date of publication of application: **25.12.2019**
(21) Application number: 19180463.2
(22) Date of filing: 17.06.2019
(51) Int. Cl.: G01V 1/50, E21B 47/00

(54) **METHOD AND SYSTEM OF LIGHT-WEIGHT CEMENT BOND EVALUATION BY ACOUSTIC VORTEX WAVES**

(30) Priority: 18.06.2018 US 201816011389
(71) Applicant: GOWell International, LLC, Houston, TX 77041 (US)
(72) Inventor: ZHAO, Jajun, Houston, TX 77064 (US); YANG, Qinshan, Katy, TX 77494 (US); ZHAO, Jinsong, Houston, TX 77007 (US)
(74) Representative: f & e patent

(57) **Abstract**

A method and system for inspecting light-weight cement downhole. The method may comprise inserting an inspection device into a casing. The inspection device may comprise a transducer, a centralizing module, and a telemetry module. The method may further comprise activating the transducer, wherein the transducer generates acoustic vortex waves, detecting the locations of fluid gaps, and creating a graph with an information handling system for analysis.

## Description

### BACKGROUND OF THE INVENTION

### Field of the Disclosure

This disclosure relates to a field for a downhole tool that may be capable of detecting in light-weight cement, bad interfaces between casing and light-weight cement, and/or bad interfaces between light-weight cement and a formation. Processing recorded cylindrical acoustic vortex waves may help identify properties within light-weight cement attached to casing.

### Background of the Disclosure

Downhole casing may be surrounded and/or encased by light-weight cement. It may be beneficial to evaluate the interface between the casing and the light-weight cement. Light-weight cement has been in use downhole for only about ten years. It has the advantage of not contaminating perforations because of its low-density. Further, light-weight cement helps to protect formations. Some formations are very soft, and the use of light-weight cement helps to avoid breaking the soft formations, which could lead to losing oil or ruining the water/oil connection.

One problem with light-weight cement, however, is that its low density is very close to the density of borehole fluids. Thus, light-weight cement and borehole fluids have roughly the same acoustic impedance, making it difficult to tell the difference between the two using methods currently known in the industry.

### BRIEF SUMMARY OF SOME OF THE PREFERRED EMBODIMENTS

These and other needs in the art may be addressed in embodiments by a device and method for evaluating light-weight cement bonds using acoustic vortex waves.

A method and system for inspecting light-weight cement downhole. The method may comprise inserting an inspection device into a casing. The inspection device may comprise a transducer, a centralizing module, and a telemetry module. The method may further comprise activating the transducer, wherein the transducer generates acoustic vortex waves, detecting the locations of fluid gaps, and creating a graph with an information handling system for analysis.

An inspection device may comprise a centralizing module, a transducer, an information handling system; and a micro-controller unit. The inspection device may further comprise an azimuthal receiver and a controller.

The foregoing has outlined rather broadly the features and technical advantages of the present invention in order that the detailed description of the invention that follows may be better understood. Additional features and advantages of the invention will be described hereinafter that form the subject of the claims of the invention. It should be appreciated by those skilled in the art that the conception and the specific embodiments disclosed may be readily utilized as a basis for modifying or designing other embodiments for carrying out the same purposes of the present invention. It should also be realized by those skilled in the art that such equivalent embodiments do not depart from the spirit and scope of the invention as set forth in the appended claims.

### BRIEF DESCRIPTION OF THE DRAWINGS

For a detailed description of the preferred embodiments of the invention, reference will now be made to the accompanying drawings in which:
Figure 1 illustrates an embodiment of an inspection system disposed downhole.
Figure 2 illustrates a top view of an embodiment of an inspection device downhole.
Figure 3 illustrates a top view of an embodiment of a transducer configuration.
Figure 4 illustrates an embodiment of electrical output signals generated by the transducer configuration.
Figure 5 illustrates an alternative embodiment of an inspection system with an azimuthal receiver.
Figure 6 illustrates low-frequency vortex waves created by the electrical output signals.
Figure 7 is a graph illustrating an example of the presence of aberrations in the bonding between light-weight cement and casing.
Figure 8 illustrates a side view of an embodiment of an inspection device disposed downhole near a fluid gap.
Figure 9 is a graph illustrating an example of the pressure encountered by an inspection device at the wellbore depth of a fluid gap.
Figure 10 shows an embodiment of casing eccentered within a borehole.
Figure 11 is a graph illustrating an example of pressure encountered by an inspection device in relation to an azimuthal angle.

### DETAILED DESCRIPTION OF THE PREFERRED EMBODIMENTS

The present disclosure relates to embodiments of a device and method for inspecting and detecting properties of cement attached to casing. More particularly, embodiments of a device and method are disclosed for evaluating light-weight cement bonds surrounding casing. In embodiments, an inspection device may generate acoustic vortex waves in surrounding casing and light-weight cement. More specifically, in embodiments, a 6-transducer configuration along the azimuthal direction may generate cylindrical acoustic vortex waves at low frequency.

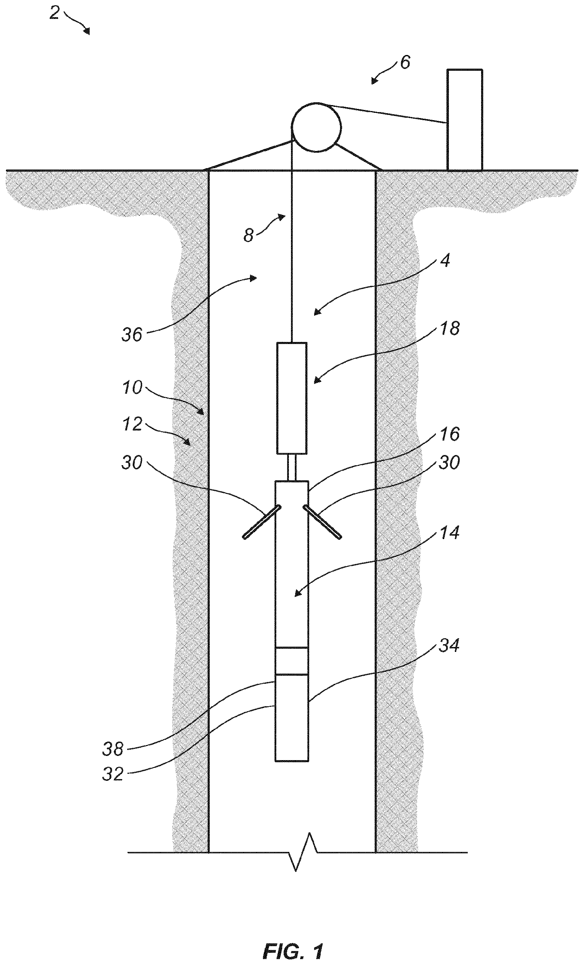

Figure 1 illustrates an inspection system 2 comprising an inspection device 4 and a service device 6. In embodiments, inspection device 4 and service device 6 may be connected by a tether 8. Tether 8 may be any suitable cable that may support inspection device 4. A suitable cable may be steel wire, steel chain, braided wire, metal conduit, plastic conduit, ceramic conduit, and/or the like. A communication line, not illustrated, may be disposed within tether 8 and connect inspection device 4 with service device 6. Without limitation, inspection system 2 may allow operators on the surface to review recorded data in real time from inspection device 4.

In embodiments, inspection device 4 may be inserted into a casing 10. In further embodiments, there may be a plurality of casing 10. Inspection device 4, as illustrated in Figure 1, may be able to determine the location of aberrations within a light-weight cement 12, which may comprise inadequate casing 10 and light-weight cement 12 adhesion, inadequate light-weight cement 12 and formation (not illustrated) adhesion, cracks in light-weight cement 12, and/or the like. In embodiments, light-weight cement 12 may have a density equal to or less than 10 pounds-per-gallon (ppg).

Figure 1 further illustrates inspection device 4 comprising a 6-transducer configuration 14, a centralizing module 16, and a telemetry module 18. In embodiments, as shown, 6-transducer configuration 14 may be disposed below centralizing module 16 and telemetry module 18. In other embodiments, not illustrated, 6-transducer configuration 14 may be disposed above and/or between centralizing module 16 and telemetry module 18.

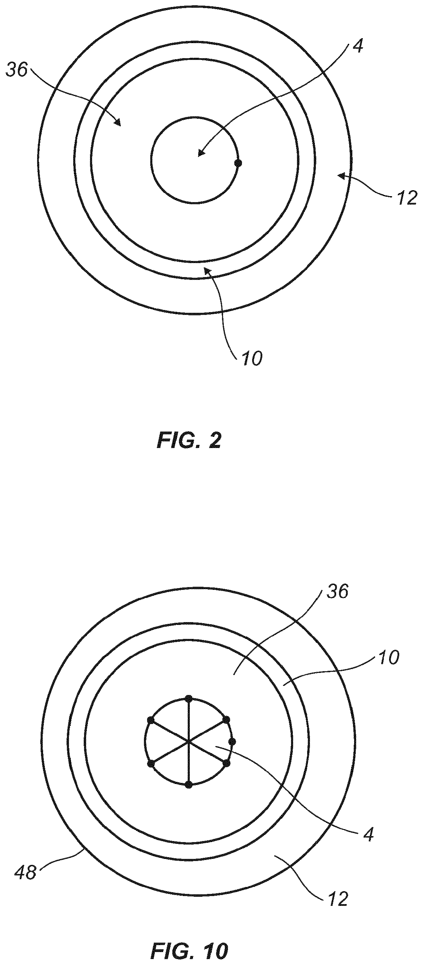

Figure 2 illustrates a top view of an embodiment of inspection device 4 downhole. Further, Figure 2 also illustrates borehole fluids 36, casing 10, light-weight cement 12, and a formation 20. In embodiments, a fluid gap 22 (not illustrated) may exist at the top of the light-weight cement 12. In embodiments, the fluid in fluid gap 22 may be water or a mixture of water and mud. Figure 2 also illustrates a detection point 23, which in embodiments, is the point where the absolute pressure is measured. In embodiments, detection point 23 may be anywhere on the perimeter of inspection device 4.

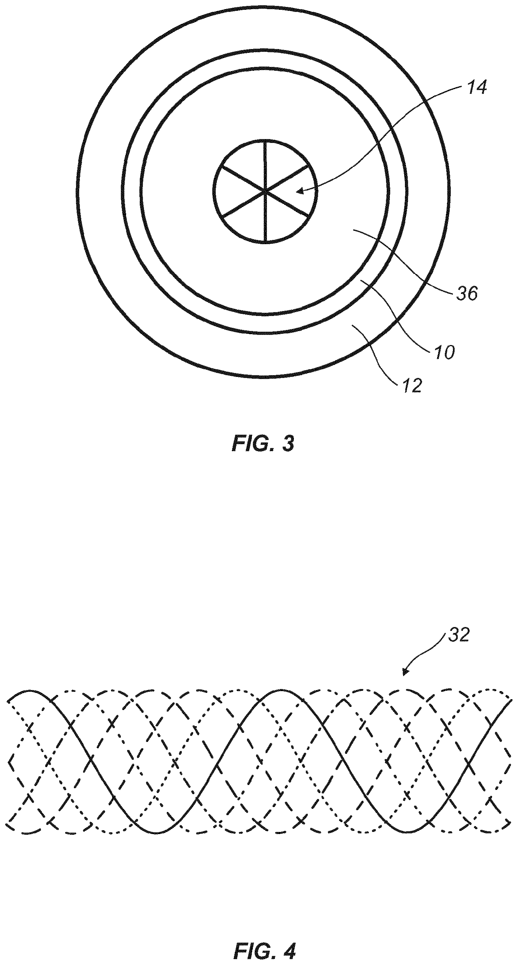

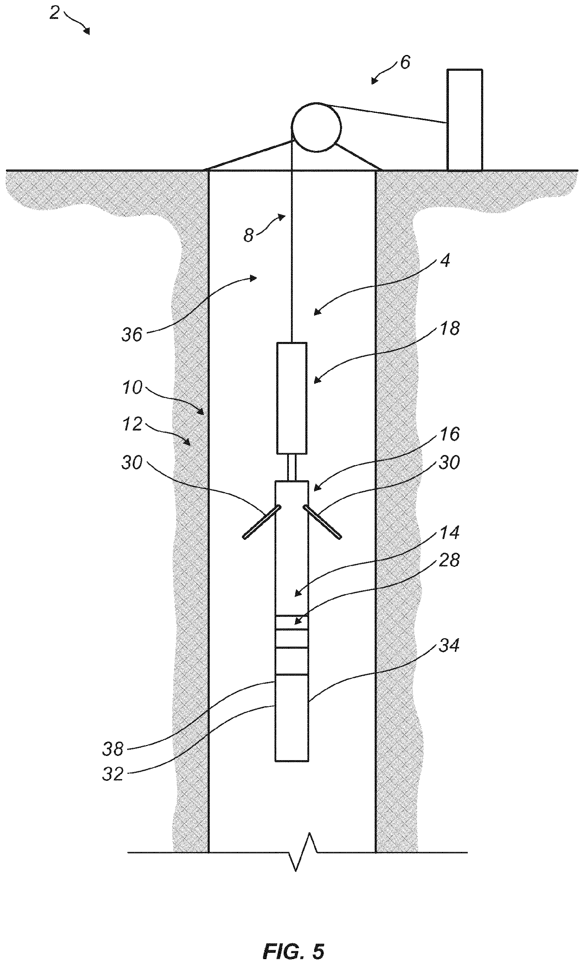

Returning to Figure 1, in embodiments, 6-transducer configuration 14 may generate cylindrical acoustic vortex waves at low frequency (less than 20 kHz). Figure 3 illustrates a top view of 6-transducer configuration 14. Alternatively, other embodiments may employ a transducer configuration with a different number of transducers. In embodiments, the 6-transducer configuration 14 is a phase-gradient transducer array along the azimuthal direction. In embodiments, the longitudinal length of 6-transducer configuration 14 may be about 300 mm to 400 mm. There is a constant phase difference covering the entire 360 degrees. Each phase difference is 60 degrees. Figure 4 illustrates electrical output signals 24 generated by 6-transducer configuration 14. In embodiments, output signals 24 each have the same amplitude and frequency. In embodiments, 6-transducer configuration 14 may be set to both generate output signals 24 and receive input signals 26. Alternatively, as illustrated in Figure 5, inspection device 4 may further comprise an azimuthal receiver 28 to receive input signals 26.

As illustrated in Figure 1, in embodiments, centralizing module 16 may be used to position inspection device 4 inside casing 10. In embodiments, centralizing module 16 laterally positions inspection device 4 at about a center of casing 10. Centralizing module 16 may be disposed at any location above and/or below 6-transducer configuration 14. In embodiments, centralizing module 16 may be disposed above 6-transducer configuration 14 and below telemetry module 18. Centralizing module 16 may comprise one or more arms 30. In embodiments, there may be a plurality of arms 30 that may be disposed at any location along the exterior of centralizing module 16. Specifically, arms 20 may be disposed on the exterior of centralizing module 16. In an embodiment, as shown, at least one arm 20 may be disposed on opposing lateral sides of centralizing module 16. Additionally, there may be at least three arms 30 disposed on the outside of centralizing module 16. Arms 30 may be moveable at about the connection with centralizing module 16, which may allow the body of arm 30 to be moved closer and/or farther away from centralizing module 16. Arms 30 may comprise any suitable material. Suitable material may be, but is not limited to, stainless steel, titanium, metal, plastic, rubber, neoprene, and/or any combination thereof. In other embodiments, not illustrated, inspection device 4 may employ a standoff instead of centralizing module 16.

Telemetry module 18, as illustrated in Figure 1, may comprise any devices and processes for making, collecting, and/or transmitting measurements. For instance, telemetry module 18 may comprise an accelerator, gyro, and the like. In embodiments, telemetry module 18 may operate to indicate where inspection device 4 may be disposed within casing 10. Telemetry module 18 may be disposed at any location above or below transducer 14. In embodiments, telemetry module 18 may send information through the communication line in tether 8 to a remote location such as a receiver or an operator in real time, which may allow an operator to know where inspection device 4 may be located within casing 10. In embodiments, telemetry module 18 may be centered about laterally in casing 10. Alternatively, in embodiments, telemetry module 18 may not be needed when data is processed downhole by inspection device 4.

As illustrated in Figure 1, a micro-controller unit 32 may be disposed within inspection device 4. In embodiments, micro-controller unit 32 may store all received, recorded, and measured data and may transmit the data in real time through a communication line in tether 8 to a remote location such as an operator on the surface. In embodiments, data may include, but not be limited to, the pressure of acoustic waves at different frequencies. Micro-controller unit 32 may comprise flash chips and/or RAM chips, which may be used to store data and/or buffer data communication. Additionally, micro-controller unit 32 may further comprise a transmitter, processing unit and/or a microcontroller. In embodiments, micro-controller unit 32 may be removed from inspection device 4 for further processing. Micro-controller unit 32 may be disposed within any suitable location on inspection device 4 such as about the top, about the bottom, or about the center of inspection device 4. In embodiments, micro-controller unit 32 may be in communication with a controller 34 by any suitable means such as a communication line.

In embodiments, an information handling system 38, discussed in further detail below, may be disposed in inspection device 4 and communicate with micro-controller unit 32 through tether 8. Information handling system 38 may analyze recorded acoustic waves, input signals 26, to evaluate the bonding of light-weight cement 12 with surrounding casing 10. In embodiments, information handling system 38 may be disposed within inspection device 4 and may transmit information through tether 8 to service device 6.

Without limitation, information handling system 38 may include any instrumentality or aggregate of instrumentalities operable to compute, classify, process, transmit, receive, retrieve, originate, switch, store, display, manifest, detect, record, reproduce, handle, or utilize any form of information, intelligence, or data for business, scientific, control, or other purposes. For example, information handling system 38 may be a personal computer, a network storage device, or any other suitable device and may vary in size, shape, performance, functionality, and price. Information handling system 38 may include random access memory (RAM), one or more processing resources such as a central processing unit (CPU) or hardware or software control logic, ROM, and/or other types of nonvolatile memory. Additional components of information handling system 38 may include one or more disk drives, one or more network ports for communication with external devices as well as various input and output (I/O) devices, such as a keyboard, a mouse, and a video display. Information handling system 38 may also include one or more buses operable to transmit communications between the various hardware components.

Controller 34, as illustrated in Figure 1, may control 6-transducer configuration 14. Controller 34 may be pre-configured at the surface to take into account the downhole logging environment and specific logging cases, which may be defined as a static configuration. It may also be dynamically configured by what 6-transducer configuration 14 may record. Controller 34 may be disposed at any suitable location on inspection device 4. In embodiments, such disposition may be about the top, about the bottom, or about the center of inspection device 4.

Service device 6 may comprise a mobile platform (e.g., a truck) or stationary platform (e.g., a rig), which may be used to lower and raise inspection device 4. In embodiments, service device 6 may be attached to inspection device 4 by tether 8. Service device 6 may comprise any suitable equipment that may lower and/or raise inspection device 4 at a set or variable speed, which may be chosen by an operator. The movement of inspection device 4 may be monitored and recorded by telemetry module 18.

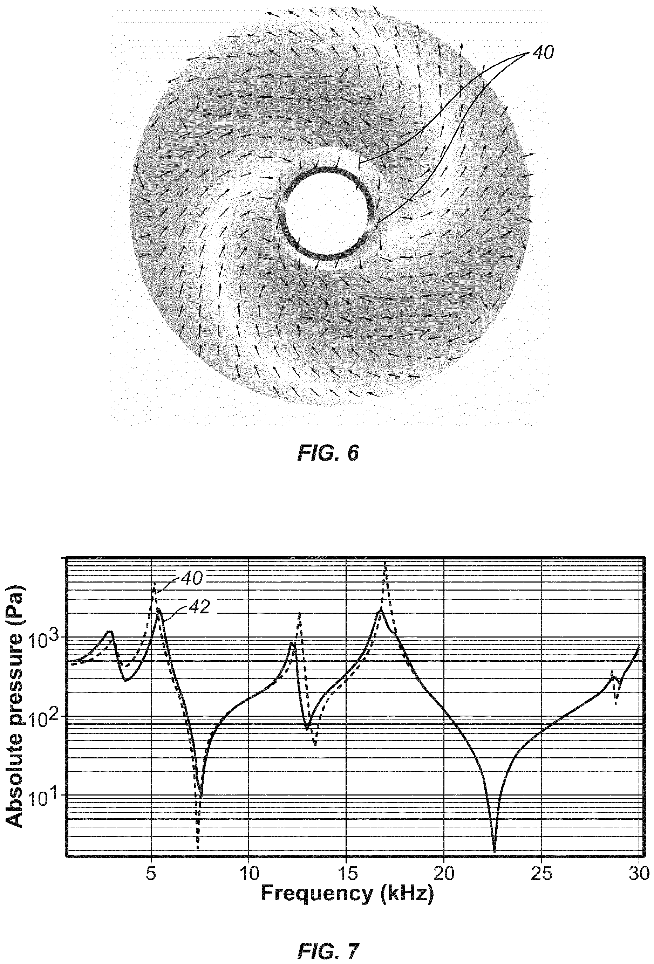

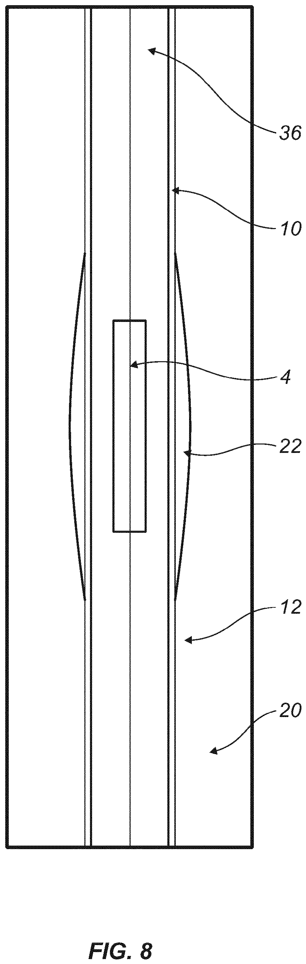

Figure 6 illustrates low-frequency vortex waves created by the electrical output signals 24. The Von Mises stress are also illustrated in Figure 6. The light color indicates the areas where the stress is low compared to the dark areas where the stress is higher. Arrows 40 represent the displacement field. In response to the electrical output signals 24, the polarization is circular rather than perpendicular. Thus, the energy created by the output signals 24 is permitted to propagate. The circular propagation allows S- and P-waves to move outward from inspection device 4 through light-weight cement 12 and into formation 20 (not illustrated). However, fluids do not support S-wave propagation. Thus, in embodiments, the energy of the output signals 24 will dissipate if the bonding between casing 10 and light-weight cement 12 does not contain aberrations such as fluid gap 22, as discussed below with Figure 8. In embodiments, if the bonding between casing 10 and light-weight cement 12 does contain aberrations, the presence of fluid gaps 22 will cause the energy of the output signals 24 to be reflected back towards inspection device 4. Further, the reflection of the energy causes higher pressures than if the energy is permitted to dissipate. Thus, higher pressures are an indication of fluid gaps 22 being present.

Figure 7 is a graph illustrating an example of the presence of aberrations, line 40, and the absence of aberrations, line 42. As seen in Figure 7, the aberrations shown by line 40 have a higher pressure than instances where there are no aberrations, line 42.

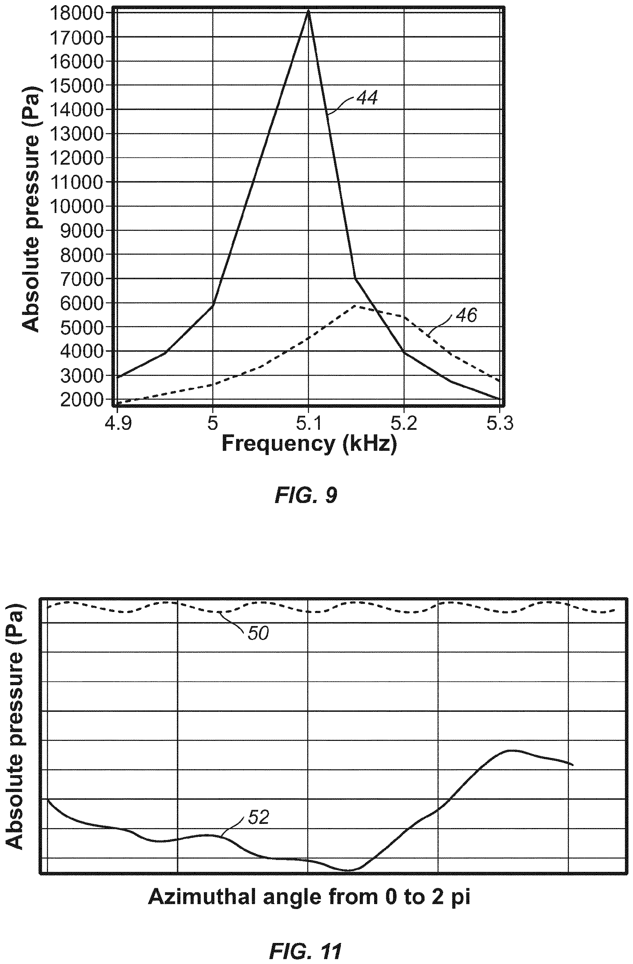

Figure 8 is a side view of inspection device 4 in the subterranean wellbore. In embodiments, inspection device 4 encounters a fluid gap 22. Figure 9 is a graph illustrating the absolute pressure encountered by inspection device 4 at the wellbore depth of the fluid gap 22. Line 44 indicates a higher pressure and thus indicates the presence of fluid gap 22. Line 46 indicates a lower pressure and thus no aberrations or fluid gaps in light-weight cement 12.

In addition to evaluating the bond between casing 10 and light-weight cement 12, inspection device 4 may also be employed to evaluate eccentricity. In embodiments, eccentricity does not affect the resonance frequency or amplitude of the vortex waves significantly. However, azimuthally detected acoustic pressure has different profiles when comparing eccentricity and fluid gaps or aberrations in light-weight cement 12. Figure 10 shows an embodiment of casing 10 within a borehole 48. Figure 11 illustrates a graph showing good bonding between casing 10 and light-weight cement 12 in a situation where casing 10 is concentric with borehole 48, line 50, and where casing 10 is eccentric with borehole 48, line 52. In embodiments, when casing 10 is concentric with borehole 48, the acoustic pressure is uniform along the azimuthal direction, while the acoustic pressure fluctuates when casing 10 is eccentric with borehole 48. This graph in Figure 11 does not indicate the thickness of light-weight cement 12. It merely shows whether casing 10 is cemented symmetrically within borehole 48.

Certain examples of the present disclosure may be implemented at least in part with non-transitory computer-readable media. For the purposes of this disclosure, non-transitory computer-readable media may include any instrumentality or aggregation of instrumentalities that may retain data and/or instructions for a period of time. Non-transitory computer-readable media may include, for example, without limitation, storage media such as a direct access storage device (e.g., a hard disk drive or floppy disk drive), a sequential access storage device (e.g., a tape disk drive), compact disk, CD-ROM, DVD, RAM, ROM, electrically erasable programmable read-only memory (EEPROM), and/or flash memory; as well as communications media such as wires, optical fibers, microwaves, radio waves, and other electromagnetic and/or optical carriers; and/or any combination of the foregoing.

Although the present invention and its advantages have been described in detail, it should be understood that various changes, substitutions and alterations may be made herein without departing from the spirit and scope of the invention as defined by the appended claims.

## Claims

1. A method for inspecting light-weight cement downhole comprising:
inserting an inspection device into a casing, wherein the inspection device comprises:
a transducer;
a centralizing module; and
a telemetry module;
activating the transducer, wherein the transducer generates acoustic vortex waves;
detecting the locations of fluid gaps; and
creating a graph with an information handling system for analysis.

2. The method of claim 1, wherein the transducer is a phase-gradient transducer array along an azimuthal direction.

3. The method of claim 2, wherein the phase-gradient transducer array comprises transducers of equal phase difference.

4. The method of any of claims 1 to 3, wherein the inspection device further comprises an azimuthal receiver.

5. The method of any of claims 1 to 4, wherein the inspection device further comprises a controller.

6. The method of any of claims 1 to 5, wherein the acoustic vortex waves are cylindrical.

7. The method of any of claims 1 to 6, wherein the acoustic vortex waves are low-frequency, such as having a frequency less than 20 kHz.

8. An inspection device comprising:
a centralizing module;
a transducer;
an information handling system; and
a micro-controller unit.

9. The device of claim 8, wherein the transducer is a phrase-gradient transducer array along an azimuthal direction.

10. The device of claim 9, wherein the phase-gradient transducer array comprises transducers of equal phase difference.

11. The device of any of claims 8 to 10, wherein the inspection device further comprises an azimuthal receiver.

12. The device of any of claims 8 to 11, wherein the inspection device further comprises a controller.

13. The device of any of claims 8 to 12, wherein the transducer generates acoustic vortex waves.

14. The device of claim 13, wherein the acoustic vortex waves are cylindrical.

15. The device of claims 13 or 14, wherein the acoustic vortex waves are low-frequency, such as having a frequency less than 20 kHz.
